# EUROPEAN PATENT APPLICATION

(11) **EP 4 603 582 A2**
(43) Date of publication of application: **20.08.2025**
(21) Application number: 25156860.6
(22) Date of filing: 16.10.2012
(51) Int. Cl.: C12N 15/09

(54) **MILK FAT GLOBULE MEMBRANE COMPONENT FOR INCREASING MUSCLE MASS**

(30) Priority: 17.10.2011 JP 2011228089
(62) Divisional of application: 12842024.7
(71) Applicant: Kao Corporation, Tokyo 103-8210 (JP)
(72) Inventor: HARAMIZU, Satoshi, Tochigi, 321-3497 (JP); MORI, Takuya, Tochigi, 321-3497 (JP); OTA, Noriyasu, Tochigi, 321-3497 (JP); SHIMOTOYODOME, Akira, Tochigi, 321-3497 (JP)
(74) Representative: Vossius & Partner Patentanwälte Rechtsanwälte mbB

(57) **Abstract**

Provided are a pharmaceutical product, a quasi-pharmaceutical product, a food, an animal food, and a material mixable thereto which have an excellent action of increasing muscle mass and are also highly safe.

An agent for increasing muscle mass comprises, as an active ingredient, a milk fat globule membrane component.

## Description

### [Field of the Invention]

The present invention relates to a pharmaceutical product, a food, and the like which exhibit an effect of increasing the muscle mass or suppressing a decrease in the muscle mass.

### [Background of the Invention]

In exercises and athletics, skeletal muscle is the most important motor organ. We do physical activities by contracting or relaxing the skeletal muscle. In addition, the skeletal muscle takes sugar and fat from the blood as energy sources for activities, and then consumes them. Thus, the skeletal muscle is also the largest energy consumption system. In addition, the skeletal muscle plays a role in the stabilization of the articulation, the maintenance of posture, protection of blood vessels and organs, and the like.

The skeletal muscle mass is kept constant as a result of the balance between the synthesis of muscle protein and the decomposition thereof, and it is unlikely that the skeletal muscle mass fluctuates drastically in daily life. However, the skeletal muscle mass and the muscle functions such as muscle strength are reduced with aging. It has been reported that, after the age of 30, our skeletal muscle is decreased at a percentage of approximately 5% every 10 years, and after the age of 60, such a reduction rate is further accelerated, and that a reduction in the skeletal muscle at a later stage of life is characterized by a selective reduction in fast muscle (type II) fibers (Non-Patent Document 1). It is said that a reduction in the skeletal muscle mass and the muscle function related to aging causes a fall resulting in injury, and also causes a reduction in the amounts of physical activities, so that it increases the risk of metabolic disorders, such as osteoporosis, obesity and insulin resistance (Non-Patent Document 2).

Moreover, a reduction in the skeletal muscle mass or a reduction in the muscle function is not only caused by aging, but these symptoms are also observed in patients suffering from congenital myasthenic syndrome. These symptoms are related to abnormal signal transduction of the neuromuscular junction that is caused by the congenital genetic mutation of molecules expressed in the neuromuscular junction (Non-Patent Document 3). In particular, Dok-7 and MuSK are considered to be molecules essential for the formation of the neuromuscular junction (Non-Patent Document 4). Furthermore, it has also been reported that neuromuscular junction formation dysfunction is caused by aging (Non-Patent Document 5).

As a means for increasing the skeletal muscle mass, the use of a steroid drug and a growth hormone has been known. However, there have been problematic side effects such as a reduction in muscle strength, myalgia, and hypoadrenalism. An increase in or maintenance of the skeletal muscle mass is considered to be desirable not only for sports lovers and athletes who aim at the improvement of their performance, but also for common people in terms of health care. Thus, it has been desired to develop a highly safe component. For example, it has been reported that a composition comprising arginine, lysine, etc. (Patent Document 1), an amino acid-containing composition (Patent Document 2), and the like have an action to increase the muscle mass.

A milk fat globule membrane (MFGM) component is a membrane-like component derived from a mammary glandular cell, which coats fat globules in milk, and it mainly consists of a phospholipid and a lipoprotein. Such a milk fat globule membrane component has a function of dispersing fat into milk. In addition to this function, the milk fat globule membrane component has also been known to have physiological functions such as an effect of increasing in and/or suppressing a decrease in a blood adiponectin level (Patent Document 3), a learning ability improving effect (Patent Document 4), and a sialomucin secretion promoting effect (Patent Document 5). In addition, recently, it has been reported that the milk fat globule membrane component increases muscle strength and improves endurance (Patent Document 6).

However, the fact that the milk fat globule membrane component has an action to increase the muscle mass, an action to suppress muscle atrophy, and an action to promote neuromuscular junction formation has been totally unknown.

### [Citation List]

### [Patent Document]

[Patent Document 1] JP-A-2009-513149
[Patent Document 2] JP-A-2008-534599
[Patent Document 3] JP-A-2007-320901
[Patent Document 4] JP-A-2007-246404
[Patent Document 5] JP-A-2007-112793
[Patent Document 6] JP-A-2010-59155

### [Non-Patent Document]

[Non-Patent Document 1] Lexell, J Neurol Sci, 84, 1988
[Non-Patent Document 2] Evans, J Gerontol A Biol Sci Med Sci, 50, 1998
[Non-Patent Document 3] Kinji OHNO, BRAIN and NERVE, 63, 2011
[Non-Patent Document 4] Osamu HIGUCHI, BRAIN and NERVE, 63, 2011
[Non-Patent Document 5] Balice-Gordon, Muscle & Nerve, 1997

### [Summary of the Invention]

The present invention relates to the following (1) to (12):
(1) An agent for increasing the muscle mass comprising, as an active ingredient, a milk fat globule membrane component.
(2) An agent for suppressing muscle atrophy comprising, as an active ingredient, a milk fat globule membrane component.
(3) An agent for promoting neuromuscular junction formation comprising, as an active ingredient, a milk fat globule membrane component.
(4) Use of a milk fat globule membrane component for the production of an agent for increasing the muscle mass.
(5) Use of a milk fat globule membrane component for the production of an agent for suppressing muscle atrophy.
(6) Use of a milk fat globule membrane component for the production of an agent for promoting neuromuscular junction formation.
(7) A milk fat globule membrane component for use in an increase in the muscle mass.
(8) A milk fat globule membrane component for use in suppression of the muscle atrophy.
(9) A milk fat globule membrane component for use in promotion of neuromuscular junction formation.
(10) A method for increasing the muscle mass, comprising administering or taking an effective amount of a milk fat globule membrane component.
(11) A method for suppressing muscle atrophy, comprising administering or taking an effective amount of a milk fat globule membrane component.
(12) A method for promoting neuromuscular junction formation, comprising administering or taking an effective amount of a milk fat globule membrane component.

### [Detailed Description of the Invention]

The present invention relates to a provision of a pharmaceutical product, a quasi-pharmaceutical product, a food, an animal food, and a material mixable thereto which have an excellent action of increasing the muscle mass, suppressing muscle atrophy, promoting neuromuscular junction formation, and the like, and are also highly safe.

The present inventors searched for an effective component for an increase in the muscle mass. As a result, the inventors found that a milk fat globule membrane component has an action to increase the muscle mass, an action to increase fast muscle fibers, an action to increase muscle protein, an action to promote neuromuscular junction formation and the like, and that this milk fat globule membrane component is useful as a pharmaceutical product, a quasi-pharmaceutical product, a food, an animal food, or the like which can exhibit effects such as an increase in the muscle mass and suppression of muscle atrophy.

The agent for increasing the muscle mass and the agent for suppressing muscle atrophy of the present invention are useful as a food, a pharmaceutical product, a quasi-pharmaceutical product, an animal food, or a material mixable thereto, which are used for an increase in the muscle mass, suppression of muscle atrophy, and promotion of neuromuscular junction formation, on daily activities for people in a wide age range including elder people.

The milk fat globule membrane component of the present invention is defined as a membrane which coats fat globules in milk, and a membrane component mixture which constitutes the aforementioned membrane. It has been known that the milk fat globule membrane component is contained much in a fraction which contains milk complex lipid such as butter milk and butter serum with high content. The milk fat globule membrane is generally composed of proteins (approximately 40 to 45%) and lipids (approximately 50 to 55%). The proteins have been known to contain a glycoprotein called milk mucin (Mather IH, Biochim Biophys Acta. (1978) 514: 25-36.) and the like. The lipids have been known to contain triglyceride and phospholipids (for example, sphingophospholipid and glycerophospholipid) in large amounts, and also contain glycosphingolipid (for example, glucosylceramide and ganglioside) (Keenan TW, Applied Science Publishers. (1983) pp. 89-130.). Furthermore, a principle example of the phospholipids is a sphingophospholipid such as sphingomyelin, and other examples include glycerophospholipids such as phosphatidyl choline and phosphatidyl ethanolamine.

The content of lipid in the milk fat globule membrane component of the present invention is preferably 10% by mass or more, more preferably 20% by mass or more, even more preferably 30% by mass or more in terms of dry matter. Also, it is preferably 100% by mass or less, more preferably 90% by mass or less, even more preferably 60% by mass or less. Thus, the content of lipid is, for example, preferably from 10 to 100% by mass, more preferably from 20 to 90% by mass, even more preferably from 30 to 60% by mass.

The content of phospholipid in the milk fat globule membrane component is preferably 5% by mass or more, more preferably 8% by mass or more, even more preferably 15% by mass or more, even more preferably 20% by mass or more in terms of dry matter. Also, it is preferably 100% by mass or less, more preferably 90% by mass or less, even more preferably 80% by mass or less, even more preferably 60% by mass or less. Thus, the content of phospholipid is preferably from 5 to 100% by mass, more preferably from 8 to 90% by mass, even more preferably from 15 to 80% by mass, even more preferably from 20 to 60% by mass.

The milk fat globule membrane component of the present invention may be prepared from milk raw material by a known method such as a centrifugation method or an organic solvent extraction method.

Examples of the milk raw material include cow milk, goat milk and the like. Among these milks, cow milk is preferably used. Furthermore, the milk raw materials include not only milk such as raw milk, nonfat milk and processed milk, but also dairy products. Examples of the dairy products include butter milk, butter oil, butter serum, whey protein concentrate (WPC) and the like. Butter milk is obtained during the production of butter granules from cream obtained by centrifugation of milk or the like. Since the milk fat globule membrane component is contained in a large amount in butter milk, such butter milk may be directly used. Likewise, since a large amount of the milk fat globule membrane component is contained in butter serum obtained during the production of butter oil, such butter serum may also be directly used.

An example of a method for preparing the milk fat globule membrane component is a method including extracting milk or daily products such as whey protein concentrate (WPC) with ether or acetone (JP-A-03-47192). Another example is a method including adjusting a pH of butter milk to an acidic range, removing protein generated by isoelectric precipitation, subjecting the resultant supernatant to a microfilter membrane treatment and drying the resultant concentrate(JP-B-3103218). Furthermore, a method including coagulating and removing protein from butter serum, then subjecting the resultant to filtration and concentration, and drying the concentrate (JP-A-2007-89535) may also be employed. According to this production method, for example, it makes possible to prepare a milk fat globule membrane component containing 20% by weight or more of complex lipid derived from milk in terms of dry matter.

The thus prepared milk fat globule membrane component may be purified by techniques such as dialysis, ammonium sulfate fractionation, gel filtration, isoelectric precipitation, ion-exchange chromatography, and solvent fractionation, as necessary, and a component whose purity has been increased by the purification may be used.

It is to be noted that the form of the milk fat globule membrane component is not particularly limited, and that the form may be any one of liquid, semisolid and solid, powdery, and the like, and any of these forms may be used alone or in combination of two or more thereof.

A milk fat globule membrane component derived from cow milk, which is commonly consumed in diet and has high purity and low price, is commercially available and is particularly preferably used. Examples of such a commercially available product include "BSCP" produced by MEGGLE JAPAN CO., LTD., "Milk Ceramide MC-5" produced by Snow Brand Milk Products Co., Ltd, "Phospholipid Concentrate" produced by New Zealand Milk Products CO., LTD and the like.

As described in the later-mentioned examples, when the milk fat globule membrane component of the present invention was administered to a mouse, it exhibited effects such as an increase in gastrocnemius muscle weight and quadriceps femoris weight, promotion of the expression of a Myh1 gene of a fast muscle fiber marker, and promotion of the expression of an IGf-1 gene associated with the synthesis of muscle protein.

Moreover, when the milk fat globule membrane component was administered to a senescence-accelerated mouse, a decrease in quadriceps femoris weight attended with accelerated senescence was suppressed. That is to say, the milk fat globule membrane component is also effective for a decrease in the muscle mass and muscle atrophy caused by aging.

Furthermore, when the milk fat globule membrane component was administered to a senescence-accelerated mouse, a decrease in the gene expression of molecules (Dok-7, MuSK) associated with neuromuscular junction formation or in proteins was suppressed. That is, the milk fat globule membrane component is also effective for a reduction in neuromuscular junction formation caused by ageing.

Accordingly, the milk fat globule membrane component of the present invention can be used as an agent for increasing the muscle mass, an agent for suppressing muscle atrophy, and an agent for promoting neuromuscular junction formation (hereinafter referred to as an "agent for increasing the muscle mass and the like"), and further, it can also be used for the production of these agents.

The use of the milk fat globule membrane component may be in a human or non-human animal or a sample derived therefrom, and it may also be either therapeutic use or non-therapeutic use. Herein, the term "non-therapeutic" is used to mean a concept excluding medical treatments, namely, a concept excluding a method for operating, treating or diagnosing a human, and more specifically, a concept excluding a method for operating, treating or diagnosing a human by a doctor or a person who receives instructions from a doctor.

In the present invention, the phrase "an increase in the muscle mass" means to increase the muscle mass, and it also includes suppression of a decrease in the muscle mass.

In addition, the term "muscle atrophy" is used to mean that muscle protein is decreased or muscle cells are decreased by the decomposition rate of the muscle protein that is higher than the synthetic rate thereof, so that the muscle mass is decreased. The muscle atrophy is broadly divided into: muscle atrophy caused by long-term bed rest or immobilization with a cast due to bone fracture, or caused by exposure to microgravity (which is referred to as "disuse muscle atrophy"); and progressive muscle atrophy caused by diseases such as amyotrophic lateral sclerosis (ALS). Moreover, the same symptoms as those of muscle atrophy may occur with aging. This muscle atrophy is referred to as sarcopenia. Accordingly, the phrase "suppression of muscle atrophy" is used to mean suppression of a decrease in the muscle mass due to inaction, aging, disease, etc.

Furthermore, the phrase "promotion of neuromuscular junction formation" is used to mean promotion of formation of a junction between motor nerve-ending and muscle fibers. Promotion of the function of neuromuscular junction is also included in this phrase.

In general, a correlation is found between muscle cross-sectional area and muscle strength (Tetsuo FUKUNAGA, "Hito no Zettai Kinryoku - Choonpa niyoru Taishi Sosei / Kinryoku no Bunseki (Human's Absolute Muscle Strength - Analysis of Appendicular Composition/Muscle Strength by Ultrasonic Wave -)," Kyorin Shoin, 1978). This shows that muscular hypertrophy occurs with an increase in muscle strength. There are many reports stating that an increase in muscle strength caused by training is larger than an increase in muscle cross-sectional area (Davis J et al. Eur J Appl Physiol 57 1988). In addition, it has also been demonstrated that, as such training progresses, muscle strength per muscle fiber cross-sectional area decreases (Ploutsz LL et al. J Appl Physiol 76 1994). It is considered that this is because, in addition to muscle cross-sectional area, the ratio of fast muscle fibers and slow twitch fibers contained in the motor nerve system or muscle has influence on the muscle strength (Komi P et al. Acta Physiol Scand 100 1997, Sale DG et al. Med Sci Sports Exerc 20 1998). Thus, muscle strength is regulated by various factors, and an increase in muscle strength and muscular hypertrophy is not necessarily a simple one-to-one relation. Accordingly, the agent for increasing the muscle mass, agent for suppressing muscle atrophy and agent for promoting neuromuscular junction formation of the present invention differ from muscle strength improvers and endurance improvers.

The agent for increasing the muscle mass and the like of the present invention, as such, may be a pharmaceutical product for humans or animals, a quasi-pharmaceutical product, a food or an animal food, which exhibits effects such as an increase in the muscle mass and suppression of muscle atrophy when it is taken or administered to animals including humans, or may also be a material or a preparation which is mixed into the aforementioned pharmaceutical product, quasi-pharmaceutical product, food or animal food and is then used.

In addition, the aforementioned food includes a food, a functional food, a food for patients, and a food for specified health use, which have such concepts as an increase in the muscle mass, an increase in fast muscle, promotion of muscle differentiation and suppression of muscle damage in people who lack exercises, middle-aged and elderly people, people on bed rest, athletes or sports lovers, and which describe such concepts, as necessary. These foods are foods which are permitted to describe their functions, and thus, they are distinguished from common foods.

The dosage forms of the aforementioned pharmaceutical products(including quasi-pharmaceutical products) may be any one of, for example, injections, suppositories, inhalation drugs, transdermal agents, various external preparations, tablets, capsules, granules, powders and syrup. Moreover, the administration form may also be either oral administration (internal use) or parenteral administration (external use, injection). Of these, oral administration is preferable.

Furthermore, these preparations with various dosage forms can be prepared by using the milk fat globule membrane component of the present invention alone or by using the milk fat globule membrane component appropriately in combination with other pharmaceutically acceptable excipients, binders, extenders, disintegrants, surfactants, lubricants, dispersing agents, buffering agents, preservatives, corrigents, flavors, coating agents, carriers, diluents, medicinal ingredients other than the milk fat globule membrane component, and the like. For instance, a liquid preparation for oral administration can be prepared by a conventional method by addition of a corrigent, a buffering agent, a stabilizing agent, and the like.

In general, the content of the milk fat globule membrane component of the present invention in such a preparation for oral administration is preferably 0.05% by mass or more, more preferably 0.2% by mass or more, and preferably 30% by mass or less, more preferably 10% by mass or less. Thus, the content of the present milk fat globule membrane component is preferably from 0.05 to 30% by mass, more preferably from 0.2 to 10% by mass.

Examples of the form of the aforementioned food comprising the milk fat globule membrane component of the present invention include various foods including foods and drinks and nourishing foods, such as refreshing beverages, tea beverages, coffee beverages, fruit juice beverages, carbonated drink, juice, jelly, wafer, biscuits, bread, noodles, and sausage. In addition, other examples include a nutrient supplying composition having the same forms as the above-mentioned oral administration preparation (tablets, capsules, syrups, and the like).

Various forms of foods can be prepared by using the milk fat globule membrane component of the present invention alone or by using the milk fat globule membrane component appropriately in combination with other food materials or a solvent, a softener, an oil, an emulsifying agent, an antiseptic, a flavor, a stabilizing agent, a colorant, an antioxidant, a moisturizing agent, a thickening agent, and active ingredients other than the milk fat globule membrane component of the present invention.

Furthermore, the milk fat globule membrane component of the present invention can be prepared into the form of a nutritional composition such as an enteral nutrient as a food for patients, for examples, a food for aged persons or patients who need bed rest, who have difficulty in taking an adequate amount of nutrients.

Further, examples of the animal foods include: feeds for small animals such as rabbits, rats, and mice; and feeds like pet foods for dogs, cats and the like. Such a feed can be prepared into the same forms as the above-mentioned food.

In general, the content of the milk fat globule membrane component of the present invention in such a food or feed is preferably 0.05% by mass or more, more preferably 0.2% by mass or more, and preferably 20% by mass or less, more preferably 5% by mass or less. Thus, the content of the present milk fat globule membrane component is preferably from 0.05 to 20% by mass, more preferably from 0.2 to 5% by mass.

The amount of intake of the agent for increasing the muscle mass and the like according to the present invention differs depending on the dosage forms and uses, but the daily dosage for an adult individual of the milk fat globule membrane component of the present invention is set at 0.1 mg or more/60 kg body weight, preferably 1 mg or more/60 kg body weight, more preferably 5 mg or more/60 kg body weight, even more preferably 100 mg or more/60 kg body weight, even more preferably 500 mg or more/60 kg body weight. Also, it is set at 5000 mg or less/60 kg body weight, preferably 3000 mg or less/60 kg body weight, more preferably 2000 mg or less/60 kg body weight, even more preferably 1000 mg or less/60 kg body weight. It is 0.1 to 5000 mg/60 kg body weight, preferably 1 to 5000 mg/60 kg body weight, more preferably 100 to 5000 mg/60 kg body weight, even more preferably 100 to 3000 mg/60 kg body weight, even more preferably 500 to 3000 mg/60 kg body weight. The subject who is administered with or takes the milk fat globule membrane component of the present invention is not particularly limited, as long as it is a human who needs or desires to be administered with or takes it. Examples of such a subject include patients with sarcopenia, humans with age-related sarcopenia, and people at risk of these diseases.

More specific examples of the subject include: sports lovers and athletes, persons with locomotive syndrome, persons with age-related sarcopenia, persons with nervous/muscle diseases (inflammatory muscle diseases, myopathy associated with medical diseases, muscular dystrophy, congenital myopathy, mitochondrial encephalomyopathy, diabetes, and the like), persons who are healthy but lack exercises in daily life because they are busy with business or household affairs, persons who are healthy but suffer from lack of muscle strength in daily life, persons who need bed rest, and persons who undergo rehabilitation training in the prognosis of surgical/medical diseases. Other examples also include persons who do not perform exercises for improvement or maintenance of the muscle mass or muscle strength in daily life or persons who lack such exercises.

Herein, sports lovers and athletes refer to persons who congenitally or a posteriori have characteristics required for physical exercises or sports, such as strength, agility and endurance. In particular, the term "sports lovers and athletes" used herein refers to professional sports athletes, and even amateur athletes who belong to a sport club or the like and aim to participate in competition and the like. In addition, persons who suffer from lack of muscle strength in daily life refer to persons who find difficulty in continuing activities in daily life, such as climbing steps in a house or at a station or walking to a supermarket for shopping. Moreover, exercises in daily life mean physical activities in addition to daily routine work, and the purpose of performing such exercises is to improve systemic health conditions and physical force. It is to be noted that such exercises do not include light activities (basic activities) in daily life, such as standing up, walking, or lifting a light article up. A person who conducts only basic activities is considered to be a person who does not perform exercises.

Herein, lack of exercises in daily life may be a condition in which the amounts of exercises recommended by U.S. CDC (Centers for Disease Control and Prevention) are not fulfilled. For instance, in "2008 Physical Activity Guidelines for Americans", the U.S. CDC has recommended the following (A) to (C) as amounts of exercises, which an adult (17 to 64 years old) should perform for a week:
(A) a middle level of aerobic activity (namely, fast walking) for 2 hours 30 minutes (150 minutes) every week, and muscle strength reinforcing activity by weight training for 2 or more days a week, which acts on all major muscle groups (leg, hip, back, abdomen, chest, shoulder, and arm); or
(B) a high level of aerobic activity (namely, jogging or running) for 1 hour 15 minutes (75 minutes) every week, and muscle strength reinforcing activity by weight training for 2 or more days a week, which acts on all major muscle groups (leg, hip, back, abdomen, chest, shoulder, and arm); or
(C) a combination of a middle level and a high level of aerobic activities for almost the same amount as described above, and muscle strength reinforcing activity by weight training for 2 or more days a week, which acts on all major muscle groups (leg, hip, back, abdomen, chest, shoulder, and arm).

With regard to the aforementioned embodiments, the present invention further discloses the following aspects:
<1> An agent for increasing the muscle mass comprising, as an active ingredient, a milk fat globule membrane component.
<2> An agent for suppressing muscle atrophy comprising, as an active ingredient, a milk fat globule membrane component.
<3> An agent for promoting neuromuscular junction formation comprising, as an active ingredient, a milk fat globule membrane component.
<4> Use of a milk fat globule membrane component for the production of an agent for increasing the muscle mass.
<5> Use of a milk fat globule membrane component for the production of an agent for suppressing muscle atrophy.
<6> Use of a milk fat globule membrane component for the production of an agent for promoting neuromuscular junction formation.
<7> A milk fat globule membrane component for use in an increase in the muscle mass.
<8> A milk fat globule membrane component for use in suppression of muscle atrophy.
<9> A milk fat globule membrane component for use in promotion of neuromuscular junction formation.
<10> A method for increasing the muscle mass, comprising administering or taking an effective amount of milk fat globule membrane component.
<11> A method for suppressing muscle atrophy, comprising administering or taking an effective amount of milk fat globule membrane component.
<12> A method for promoting neuromuscular junction formation, comprising administering or taking an effective amount of milk fat globule membrane component.
<13> In <2>, <5>, <8> and <11> above, the suppression of muscle atrophy is suppression of muscle atrophy related to aging.
<14> In <3>, <6>, <9> and <12> above, the promotion of neuromuscular junction formation is suppression of neuromuscular junction formation dysfunction related to aging.
<15> In <4> to <6> above, the use is non-therapeutic use.
<16> In <10> to <12> above, the method is a non-therapeutic method.
<17> In <10> to <12> above, the subject who is administered with or takes the milk fat globule membrane component is an animal or a human who needs or desires an increase in the muscle mass, an animal or a human who needs or desires suppression of muscle atrophy, or an animal or a human who needs or desires promotion of neuromuscular junction formation.
<18> With regard to the agent for increasing the muscle mass according to <1> and <4> above, the agent for suppressing muscle atrophy according to <2> and <5> above, and the agent for promoting neuromuscular junction formation according to <3> and <6> above, the form of each agent is a food, and preferably a functional food.
<19> Use of a milk fat globule membrane component in a functional food for an increase in the muscle mass.
<20> Use of a milk fat globule membrane component in a functional food for suppression of muscle atrophy.
<21> Use of a milk fat globule membrane component in a functional food for promotion of neuromuscular junction formation.
<22> The use of the milk fat globule membrane component according to <20> above, wherein the suppression of muscle atrophy is suppression of muscle atrophy related to aging.
<23> The use of the milk fat globule membrane component according to <21> above, wherein the promotion of neuromuscular junction formation is suppression of neuromuscular junction formation dysfunction related to aging.
<24> In <19> to <23> above, wherein the use is non-therapeutic use.
<25> The milk fat globule membrane component according to <7> to <9> above, wherein the use is non-therapeutic use.

Hereinafter, production examples, test examples and formulation examples will be described as representative examples of the present invention.

### [Examples]

Test Example 1: Action of milk fat globule membrane component to increase the muscle mass

The action of the milk fat globule membrane component to increase the muscle mass was evaluated as follows.

As a milk fat globule membrane component, BSCP produced by MEGGLE JAPAN Co., LTD. was used. After completion of preliminary feeding for 1 week, 7-week-old C57BL/6J mice were divided into three groups (a control group, an exercise group, and a milk fat globule membrane component/exercise group) (6 mice per group), such that the body weights of mice became equal in each group. After completion of the grouping, the mice in the control group and in the exercise group were fed with a control diet (30% lipid, 20% casein, 13% sucrose, 28.5% potato starch, 4% cellulose, 1% vitamin (trade name: Vitamin-Mixed AIN-76, Oriental Bioservice, Inc.), 3.5% mineral (trade name: Mineral-Mixed AIN-76, Oriental Bioservice, Inc.) for 20 weeks. On the other hand, the mice in the milk fat globule membrane component/exercise group were fed with a test diet containing the milk fat globule membrane component (30% lipid, 20% casein, 13% sucrose, 28.5% potato starch, 4% cellulose, 1% vitamin, 3.5% mineral, and 1% milk fat globule membrane component) for 20 weeks. During the test period, the mice in the exercise group and in the milk fat globule membrane component/exercise group were subjected to exercise stress (15-20 m/min, 30 minutes/single exercise, three times per week) with a treadmill (MUROMACHI KIKAI Co., LTD.; MK-680). After completion of the test, the mice were anesthetized by sevoflurane inhalation, while feeding under resting conditions. Thereafter, gastrocnemius muscle and quadriceps femoris were collected from each mouse, and the weights thereof were then measured. The muscle weight is shown in Table 1.

**[Table 1]**

| Muscle weight measured at anatomy | | | |
|---|---|---|---|
| | Control group | Exercise group | Milk fat globule membrane component/exercise group |
| Gastrocnemius muscle weight (mg) | 282±20 | 315±8 | 335±4*$ |
| Quadriceps femoris weight (mg) | 355±2 9 | 399±26 | 426±11* |

| | | | |
|---|---|---|---|
| Statistically significant difference to control group: * *P* < 0.05 (Fisher test) Statistically significant difference to exercise group: $ *P* < 0.05 (Fisher test) | | | |

The gastrocnemius muscle weight measured at anatomy in the mice of the milk fat globule membrane component/exercise group was significantly higher than those in the mice of the control group and the exercise group. The quadriceps femoris weight measured at anatomy in the mice of the milk fat globule membrane component/exercise group was also significantly higher than that in the mice of the control group. Accordingly, it became clear in the present test that the milk fat globule membrane component has an action to increase the muscle mass.

Test Example 2: Action of milk fat globule membrane component to promote muscle differentiation

The action of the milk fat globule membrane component to promote energy consumption and lipid combustion was evaluated as follows.

As a milk fat globule membrane component, BSCP produced by MEGGLE JAPAN Co., LTD. was used. After completion of preliminary feeding for 1 week, 7-week-old C57BL/6J mice were divided into three groups (a control group, an exercise group, and a milk fat globule membrane component/exercise group) (6 mice per group), such that the body weights of mice became equal in each group. After completion of the grouping, the mice in the control group and in the exercise group were fed with a control diet (30% lipid, 20% casein, 13% sucrose, 28.5% potato starch, 4% cellulose, 1% vitamin (trade name: Vitamin-Mixed AIN-76, Oriental Bioservice, Inc.), 3.5% mineral (trade name: Mineral-Mixed AIN-76, Oriental Bioservice, Inc.)) for 20 weeks. On the other hand, the mice in the milk fat globule membrane component/exercise group were fed with a test diet containing the milk fat globule membrane component (30% lipid, 20% casein, 13% sucrose, 28.5% potato starch, 4% cellulose, 1% vitamin, 3.5% mineral, and 1% milk fat globule membrane component) for 20 weeks. During the test period, the mice in the exercise group and in the milk fat globule membrane component/exercise group were subjected to exercises stress (15-20 m/min, 30 minutes/single exercise, three times per week) with a treadmill (MUROMACHI KIKAI Co., LTD.; MK-680). After completion of the test, the mice were anesthetized by sevoflurane inhalation, while feeding under resting conditions. Thereafter, gastrocnemius muscle was collected from each mouse.

Using RNeasy Fibrous Tissue Mini Kit (Qiagen), RNA samples were obtained from the collected gastrocnemius muscle. Each RNA sample was quantified, the amount of RNA for a single reaction was then set at 125 ng, and a reverse transcription reaction was then carried out in a reaction solution (Reverse Transcriptase XL (TAKARA BIO INC.), 1 x PCR buffer II (Applied Biosystems Inc.), 5 mM MgCl₂, 1 mM dNTP mix, 2.5 µM Oligo d [T]₁₈ (New England Biolabs Inc.), and 1 U/ml RNase inhibitor (TAKARA BIO INC.)), so as to obtain cDNA. The reaction conditions were at 42°C for 10 minutes, at 52°C for 30 minutes, and at 99°C for 5 minutes.

Using the obtained cDNA as a template, quantitative PCR was carried out employing ABI PRISM 7700 Sequence Detector (Applied Biosystems Inc.). The obtained expression level was corrected using the expression level of 36B4 mRNA as a reference, and represented as a relative mRNA expression level when the control group was defined as 1. The following primers were used: IGF-1 (NM_010512, Forward: AGTTCGTGTGTGGACCGAGG (SEQ ID NO: 1), Reverse: CAGCTCCGGAAGCAACACTC (SEQ ID NO: 2)), Atrogin-1 (NM_026346, Forward: GCAGCAGCTGAATAGCATCCA (SEQ ID NO: 3), Reverse: GGTGATCGTGAGGCCTTTGAA (SEQ ID NO: 4)), MuRF1(NM_001039048, Forward: AATGTAGAAGCCTCCAAGGGC (SEQ ID NO: 5), Reverse: CTGTCCCAAAGTCAATGGCC (SEQ ID NO: 6)), and Myh1(NM_030679, Forward: GCTAGTAACATGGAGGTCA (SEQ ID NO: 7), Reverse: TAAGGCACTCTTGGCCTTTATC (SEQ ID NO: 8)). The results are shown in Table 2.

**[Table 2]**

| Expression of IGF1, Atrogin-1, MuRF1 and Myh1 genes | | | |
|---|---|---|---|
| | Control group | Exercise group | Milk fat globule/exercise group |
| IGF-1 | 1.00±0.07 | 1.44±0.17 | 1.63±0.09* |
| Atrogin-1 | 1.00±0.11 | 0. 80±0.08 | 0.66±0.10* |
| MuRF1 | 1.00±0.03 | 0.75±0.06 | 0.76±0.04* |
| Myh1 | 1.00±0.07 | 1.29±0.13 | 1.55±0.12* |

| | | | |
|---|---|---|---|
| Statistically significant difference to control group: * *P* < 0.05 (Fisher test) | | | |

The expression levels of the gastrocnemius muscle IGF-1 gene and Myh1 gene in the mice of the milk fat globule/exercise group were significantly higher than those in the mice of the control group. In contrast, the expression levels of the Atrogin-1 gene and MuRF1 gene in the mice of the milk fat globule/exercise group were significantly lower than those in the mice of the control group. As IGF-1 plays an important role in the synthesis of muscle protein, and thus it was clarified that the milk fat globule membrane component has an action to promote the synthesis of muscle protein. Moreover, since Myh1 is a fast muscle fiber marker, it was also clarified in the present test that the milk fat globule membrane component has an action to increase fast muscle. Furthermore, since Atrogin-1 and MuRF1 increase decomposition of muscle protein, it was clarified that the milk fat globule membrane component has an action to suppress decomposition of muscle protein.

Test Example 3: Action of milk fat globule membrane component to suppress muscle atrophy examined using aging model mice

The action of the milk fat globule membrane component on muscle atrophy and neuromuscular junction formation was evaluated as follows.

### 1) Total muscle weight

As a milk fat globule membrane component, BSCP produced by MEGGLE JAPAN Co., LTD. was used. 24-week-old SAMP1 (senescence-accelerated mice) and ICR mice were divided into three groups (an ICR control group, a SAMP1 control group, and a SAMP1 milk fat globule membrane component group) (6-8 mice per group). After completion of the grouping, the mice in the ICR control group and in the SAMP1 control group were fed with a control diet (5.5% lipid, 6% casein, 75.6% potato starch, 8.4% cellulose, 1% vitamin (trade name: Vitamin-Mixed AIN-76, Oriental Bioservice, Inc.), and 3.5% mineral (trade name: Mineral-Mixed AIN-76, Oriental Bioservice, Inc.) for 20 weeks. On the other hand, the mice in the SAMP1 milk fat globule membrane component group were fed with a test diet containing the milk fat globule membrane component (5.5% lipid, 5.5% casein, 75.1% potato starch, 8.4% cellulose, 1% vitamin, 3.5% mineral, 1% milk fat globule membrane component) for 20 weeks. During the test period, the SAMP1 mice were free to conduct locomotor activity in a rotating cage. After completion of the test, the mice were anesthetized by sevoflurane inhalation, while feeding under resting conditions. Thereafter, extensor digitorum longus muscle, musculus plantaris, and quadriceps femoris were collected, and the total weight of these muscles was measured. The total muscle weight is shown in Table 3.

**[Table 3]**

| | ICR control group | SAMP1 control group | SAMP1 milk fat globule group |
|---|---|---|---|
| Total muscle weight (mg) | 497±10* | 370±10 | 406±7* |

| | | | |
|---|---|---|---|
| Statistically significant difference to SAMP1 control group: * *P* < 0.05 (Fisher test) | | | |

The total muscle weight of the SAMP1 control group was significantly lower than that of the ICR control group. Thus, it is found that the muscle has shrunk due to aging. In addition, the total muscle weight of the SAMP1 milk fat globule membrane component group was significantly higher than that of the SAMP1 control group. Accordingly, it was clarified that the milk fat globule membrane component has an action to suppress muscle atrophy.

### 2) Gene expression

Using RNeasy Fibrous Tissue Mini Kit (Qiagen), RNA samples were obtained from quadriceps femoris. Each RNA sample was quantified, the amount of RNA for a single reaction was then set at 125 ng, and a reverse transcription reaction was then carried out in a reaction solution (Reverse Transcriptase XL (TAKARA BIO INC.), 1 x PCR buffer II (Applied Biosystems Inc.), 5 mM MgCl₂, 1 mM dNTP mix, 2.5 µM Oligo d [T]₁₈ (New England Biolabs Corporation), and 1 U/ml RNase inhibitor (TAKARA BIO INC.)), so as to obtain cDNA. The reaction conditions were at 42°C for 10 minutes, at 52°C for 30 minutes, and at 99°C for 5 minutes.

Using the obtained cDNA as a template, quantitative PCR was carried out employing ABI PRISM 7700 Sequence Detector (Applied Biosystems Inc.). The obtained expression level was corrected using the expression level of 36B4 mRNA as a reference, and represented as a relative mRNA expression level when the control group was defined as 1. The following primers were used: Dok-7 (NM_172708, Forward: TGAGCTTCCTGTTTGACTGCA (SEQ ID NO: 9), Reverse: GCAACACGCTCTTCTGAGGC (SEQ ID NO: 10)), and MuSK (NM_001037130, Forward: CATGGCAGAGTTTGACAACCC (SEQ ID NO: 11), Reverse: TTCGGAGGAACTCATTGAGGTC (SEQ ID NO: 12)). Relative values obtained when the gene expression of the ICR control group was defined as 1 are shown in Table 4.

**[Table 4]**

| | ICR control group | SAMP1 control group | SAMP1 milk fat globule group |
|---|---|---|---|
| Dok-7 | 1.00±0.05* | 0.72±0.02 | 0.95±006* |
| MuSK | 1.00±0.03 | 1.00±0.06 | 1.27±0.06* |

| | | | |
|---|---|---|---|
| Statistically significant difference to SAMP1 control group: * *P* < 0.05 (Fisher test) | | | |

The expression level of the Dok-7 gene in the SAMP1 control group was significantly lower than that in the ICR control group. Thus, it is found that the expression of the Dok-7 gene was decreased due to aging. In addition, the expression levels of the Dok-7 and MuSK genes in the SAMP1 milk fat globule membrane component group were significantly higher than those in the SAMP1 control group. Accordingly, it was clarified that the milk fat globule membrane component has an action to suppress neuromuscular junction formation dysfunction and an action to promote neuromuscular junction formation.

### 3) Protein expression

Using Polytron, quadriceps femoris was homogenized in CelLytic^{™} MT Mammalian Tissue Lysis/Extraction Reagent (SIGMA Corporation). The homogenate was centrifuged at 15,000 rpm at 4°C for 15 minutes, and the obtained supernatant was used as a protein lysis sample. After quantification of the protein, the concentration of the protein was adjusted to be 1 ug/uL using CelLytic^{™} MT Mammalian Tissue Lysis/Extraction Reagent and 4 × SDS sample buffer (Novagen Inc.). 10 uL of a supernatant obtained by heat denaturation (100°C, 5 min) and centrifugation at 5,000 rpm at 4°C for 5 minutes was subjected to SDS-PAGE. After completion of the SDS-PAGE, the protein was transcribed on a PVDF membrane (Millipore Corporation) (200 mA, 1.5 hr), and the membrane was then immersed in PVDF blocking Reagent (TOYOBO Co., Ltd.). After completion of the blocking operation, the membrane was immersed at O/N in antibody-diluted solution 1 (Immunoreaction Enhancer solution 1, TOYOBO Co., Ltd.) prepared by dilution (1 : 1000) of a primary antibody against MuSK (ab92950, Abcam), Dok-7 (AF6298, R & D) and α-tubulin (2144S, Cel Signaling). The resulting membrane was washed with T-TBS several times (four or more times), and was then immersed in antibody-diluted solution 2 (Immunoreaction Enhancer solution 2, TOYOBO Co., Ltd.) prepared by dilution of a HRP-labeled antibody against each antigen. The resulting membrane was washed with T-TBS several times (six or more times), and a protein of interest was then detected and quantified with ECL Prime Western Blotting Detection System (GE Healthcare Corporation) and a chemiluminescence detector (ChemiDoc XRS, Bio-Rad Laboratories Inc.). The following antibodies and antibody dilution magnifications were applied in the present test. Relative values obtained when the protein expression of the ICR control group was defined as 1 are shown in Table 5.

**[Table 5]**

| | ICR control group | SAMP1 control group | SAMP1 milk fat globule group |
|---|---|---|---|
| Dok-7 | 1.00±0.05 | 0.93±0.02 | 1.21±0.06* |
| MuSK | 1.00±0.12* | 0.60±0.05 | 0.71±0.03* |

| | | | |
|---|---|---|---|
| Statistically significant difference to SAMP1 control group: * *P* < 0.05 (Fisher test) | | | |

The expression level of the Dok-7 protein gene in the SAMP1 milk fat globule membrane component group was significantly higher than that in the SAMP1 control group. In addition, the expression level of the MuSK protein in the SAMP1 control group was significantly lower than that in the ICR control group, and thus, it is found that the expression of the MuSK protein was decreased due to aging. Moreover, the expression level of the MuSK protein gene in the SAMP1 milk fat globule membrane component group was significantly higher than that in the SAMP1 control group. Accordingly, it was clarified that the milk fat globule membrane component has an action to suppress neuromuscular junction formation dysfunction and an action to promote neuromuscular junction formation.
The present invention is characterized by the following preferred items:
1. An agent for increasing muscle mass comprising, as an active ingredient, a milk fat globule membrane component.
2. An agent for suppressing muscle atrophy comprising, as an active ingredient, a milk fat globule membrane component.
3. An agent for promoting neuromuscular junction formation comprising, as an active ingredient, a milk fat globule membrane component.
4. The agent for suppressing muscle atrophy according to item 2, which suppresses muscle atrophy related to aging.
5. The agent for promoting neuromuscular junction formation according to item 3, which suppresses neuromuscular junction formation dysfunction related to aging.
6. Use of a milk fat globule membrane component for the production of an agent for increasing muscle mass.
7. Use of a milk fat globule membrane component for the production of an agent for suppressing muscle atrophy.
8. Use of a milk fat globule membrane component for the production of an agent for promoting neuromuscular junction formation.
9. The use according to item 7, wherein the suppression of muscle atrophy is suppression of muscle atrophy related to aging.
10. The use according to item 8, wherein the promotion of neuromuscular junction formation is suppression of neuromuscular junction formation dysfunction related to aging.
11. A milk fat globule membrane component for use in an increase in muscle mass.
12. A milk fat globule membrane component for use in suppression of muscle atrophy.
13. A milk fat globule membrane component for use in promotion of neuromuscular junction formation.
14. The milk fat globule membrane component according to item 12, wherein the suppression of muscle atrophy is suppression of muscle atrophy related to aging.
15. The milk fat globule membrane component according to item 13, wherein the promotion of neuromuscular junction formation is suppression of neuromuscular junction formation dysfunction related to aging.
16. A method for increasing muscle mass, comprising administering or taking an effective amount of milk fat globule membrane component.
17. A method for suppressing muscle atrophy, comprising administering or taking an effective amount of milk fat globule membrane component.
18. A method for promoting neuromuscular junction formation, comprising administering or taking an effective amount of milk fat globule membrane component.
19. The method according to item 17, which suppresses muscle atrophy related to aging.
20. The method according to item 18, which suppresses neuromuscular junction formation dysfunction related to aging.
21. The method according to items 16 to 20, which is a non-therapeutic method.

## Claims

1. A milk fat globule membrane component for use in an increase in muscle mass.

2. A milk fat globule membrane component for use in suppression of muscle atrophy.

3. A milk fat globule membrane component for use in promotion of neuromuscular junction formation.

4. The milk fat globule membrane component for use according to claim 2, wherein the suppression of muscle atrophy is suppression of muscle atrophy related to aging.

5. The milk fat globule membrane component for use according to claim 3, wherein the promotion of neuromuscular junction formation is suppression of neuromuscular junction formation dysfunction related to aging.

6. An agent for use in increasing muscle mass comprising, as an active ingredient, a milk fat globule membrane component.

7. An agent for use in suppressing muscle atrophy comprising, as an active ingredient, a milk fat globule membrane component.

8. An agent for use in promoting neuromuscular junction formation comprising, as an active ingredient, a milk fat globule membrane component.

9. The agent for use in suppressing muscle atrophy according to claim 7, which suppresses muscle atrophy related to aging.

10. The agent for use in promoting neuromuscular junction formation according to claim 8, which suppresses neuromuscular junction formation dysfunction related to aging.

11. A non-therapeutic method for increasing muscle mass, comprising administering or taking an effective amount of milk fat globule membrane component.

12. A non-therapeutic method for suppressing muscle atrophy, comprising administering or taking an effective amount of milk fat globule membrane component.

13. A non-therapeutic method for promoting neuromuscular junction formation, comprising administering or taking an effective amount of milk fat globule membrane component.

14. The non-therapeutic method according to claim 12, which suppresses muscle atrophy related to aging.

15. The non-therapeutic method according to claim 13, which suppresses neuromuscular junction formation dysfunction related to aging.
